(19) Europäisches Patentamt
European Patent Office
Office européen des brevets

(11) Publication number: **0 312 360 B1**

(12) **EUROPEAN PATENT SPECIFICATION**

(45) Date of publication of patent specification: **17.06.92**

(51) Int. Cl.[5]: **C07C 401/00**, C07B 59/00, G01N 33/82

(21) Application number: **88309609.1**

(22) Date of filing: **13.10.88**

# (54) 25-Hydroxy vitamin D3 derivatives.

(30) Priority: **14.10.87 JP 258584/87**
**25.08.88 JP 211364/88**

(43) Date of publication of application:
**19.04.89 Bulletin 89/16**

(45) Publication of the grant of the patent:
**17.06.92 Bulletin 92/25**

(84) Designated Contracting States:
**CH DE FR LI NL**

(56) References cited:
**WO-A-80/02562**
**FR-A- 2 376 864**
**US-A- 4 119 647**
**US-A- 4 424 161**

**JOURNAL OF BIOCHEMISTRY, vol. 98, no. 4, October 1985, pages 991-998, Tokyo, JP; I. YAMAMOTO et al.: "Monoclonal antibody for calcitriol (1alpha,25-dihydroxyvitamin D3)"**

(73) Proprietor: **TOYO JOZO KABUSHIKI KAISHA**
**632-1 Mifuku Ohito-cho**
**Tagata-gun Shizuoka-ken(JP)**

(72) Inventor: **Nakagawa, Nobuaki**
**865-8 Ohito Ohito-cho**
**Tagata-gun Shizuoka-ken(JP)**
Inventor: **Ikuta, Shigeru**
**1277-5 Kasiya Kannami-cho**
**Tagata-gun Shizuoka-ken(JP)**
Inventor: **Tanabe, Miyuki**
**No. 774 Mamiya Kannami-cho**
**Tagata-gun Shizuoka-ken(JP)**

(74) Representative: **Woods, Geoffrey Corlett et al**
**J.A. KEMP & CO. 14 South Square Gray's Inn**
**London WC1R 5EU(GB)**

EP 0 312 360 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to 25-hydroxy vitamin $D_3$ derivatives, a process for their preparation and assay methods using them.

Known assay methods of 25-hydroxy vitamin $D_3$ include a competitive protein binding assay (CPBA) method and a radio immunoassay (RIA) method. In these methods, a tritium [$^3H$] labelled compound is used (PCT/JP 56/500538), for example a 25-hydroxy vitamin $D_3$ derivative labelled with tritium at the 1-position (JP-A-60-163859). In general the vitamin D binding protein used in the CPBA method is prepared from the plasma of a rat which has been fed with vitamin D deficient feed [Vitamin, 55(12), 595 - 605 : 1981]. A compound having a side chain with a carbonyl terminal group is known as a hapten for the preparation of antibodies used in the RIA method (JP-A-58-92656, JP-A-55-47653 and JP-A-59-148775). In these assay methods tritium labelled 25-hydroxy vitamin $D_3$ is used.

Compounds labelled with tritium have low specific activity as compared with those labelled with $^{32}P$ or $^{125}I$ in terms of radiation energy, and require high cost and cumbersome procedures. Known tritium labelled vitamin $D_3$ derivatives have only a low rate of $\beta$-ray emission. There have been no reports on $^{125}I$-labelled vitamin $D_3$ derivatives, which would emit $\gamma$-rays with high energy, since the conjugated triene structure of vitamin $D_3$ is known to be unstable and thought to be subject to auto-degradation by a radical reaction.

We have found an iodine radioisotope labelled 25-hydroxy vitamin $D_3$ derivative with high radiation energy which has superior properties over tritium or $^{32}P$-labelled compounds.

The present invention provides an iodine radioisotope labelled 25-hydroxy vitamin $D_3$ derivative of formula (III):

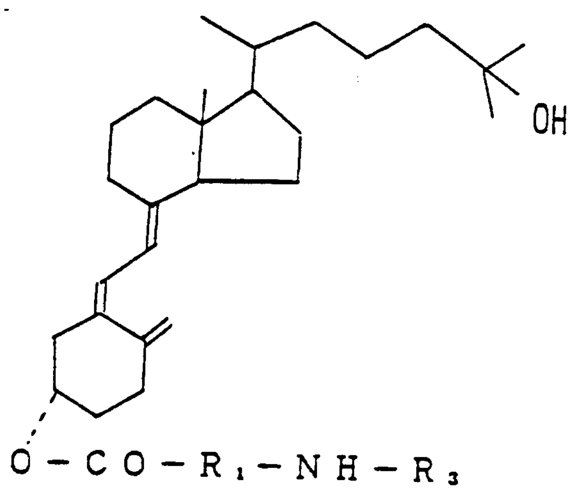

wherein $R_1$ is a $C_{1-10}$ alkylene group and $R_3$ is a group containing an iodine radioisotope.

The iodine radioisotope is preferably $^{125}I$. The vitamin $D_3$ derivative of the present invention is stable and not subject to structural degradation by $\gamma$-rays; hence it is useful for use in radio immunoassay techniques.

In general radioisotope labelling techniques, direct labelling (chloramin-T method) and indirect labelling (Bolton-Hunter reagent method) can be used to provide the radioisotope labelled compound (Amersham Note, 1 November 1981). The indirect labelling method with mild reaction conditions is preferred for preparing the derivative of formula (III) due to the instability of vitamin D against acids, oxygen, oxidizing agents, heat and light.

The derivative of formula (III) may be prepared from a 25-hydroxy vitamin $D_3$ amino acid derivative having a side chain terminated with an amino group.

The present invention therefore provides a 25-hydroxy vitamin $D_3$ amino acid derivative of formula (I):

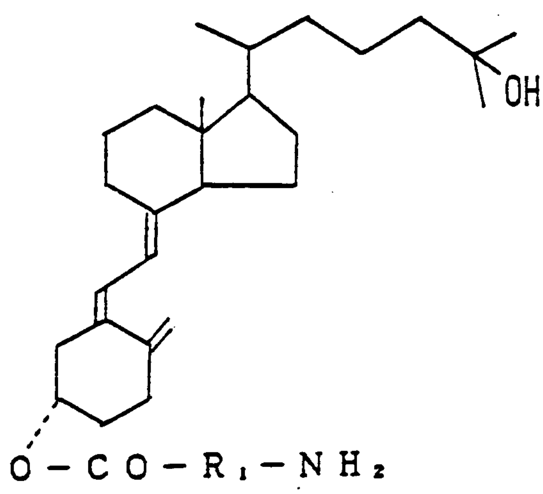

wherein $R_1$ is a $C_{1-10}$ alkylene, preferably a $C_{2-6}$ alkylene, group.

In the attached drawings:

Fig.1 : Standard curve of ($^{125}I$) labelled 25-hydroxy vitamin $D_3$ derivative;

Fig.2 : Standard curve of ($^{125}I$) or ($^{3}H$) labelled 25-hydroxy vitamin $D_3$ derivative;

Fig.3 : Standard curve of mono-iodo ($^{125}I$) or di-iodo ($^{125}I$) labelled 25-hydroxy vitamin $D_3$ derivative;

Fig.4 : Standard curve of compounds of formula (IV) (as hereinafter defined) wherein $R_1$ is ( $-(CH_2)n-$ ) wherein n = 1, n = 2 or n = 3;

Fig.5 : Standard curve of ($^{125}I$) labelled 25-hydroxy vitamin $D_3$ derivative on a CPBA-method using DBP instead of anti 25-hydroxy vitamin $D_3$ antibody; and

Fig. 6 : Stability curve of ($^{125}I$) labelled 25-hydroxy vitamin $D_3$ derivative on a CPBA-method using DBP instead of anti 25-hydroxy vitamin $D_3$ antibody.

The derivative of formula (I) may act as a hapten when it is bound to a carrier protein and inoculated into an animal to obtain antibodies. Using the antibodies and the iodine radioisotope labelled compound, we have also provided a highly sensitive and useful radio immunoassay system of 25-hydroxy vitamin $D_3$ in a specimen.

The present invention also provides an assay method of 25-hydroxy vitamin $D_3$ in a specimen which comprises:

adding, to a specimen containing 25-hydroxy vitamin $D_3$ to be assayed, an iodine radioisotope labelled 25-hydroxy vitamin $D_3$ derivative of formula (III) as defined above;

adding anti 25-hydroxy vitamin $D_3$ antibodies to the specimen to bind competitively with the 25-hydroxy vitamin $D_3$ and the iodine radioisotope labelled 25-hydroxy vitamin $D_3$ derivative of formula (III);

separating the thus produced iodine radioisotope labelled 25-hydroxy vitamin $D_3$ derivative-anti 25-hydroxy vitamin $D_3$ antibody complex from the iodine radioisotope labelled 25-hydroxy vitamin $D_3$ derivative; and

measuring the amount of the iodine radioisotope label in bound or free form.

The iodine radioisotope labelled compound of the present invention can also be used in a CPBA method using DPB (vitamin D binding protein) for assaying 25-hydroxy vitamin $D_3$ with high sensitivity.

The present invention also provides an assay method of 25-hydroxy vitamin $D_3$ in a specimen which comprises:

adding, to a specimen containing 25-hydroxy vitamin $D_3$ to be assayed, an iodine radioisotope labelled 25-hydroxy vitamin $D_3$ derivative of formula (III) as defined above;

adding vitamin D binding protein to the specimen to bind competitively with the 25-hydroxy vitamin $D_3$ and the iodine radioisotope labelled 25-hydroxy vitamin $D_3$ derivative of formula (III);

separating the thus produced iodine radioisotope labelled 25-hydroxy vitamin $D_3$ derivative-vitamin D binding protein complex from the iodine radioisotope labelled 25-hydroxy vitamin $D_3$ derivative; and

measuring the amount of the iodine radioisotope in bound or free form.

The 25-hydroxy vitamin $D_3$ amino acid derivative of formula (I) can be obtained by reacting an amino acid with the 3$\beta$-hydroxy group of 25-hydroxy vitamin $D_3$. The compound of formula (I) can be used as a hapten in a preparation of anti 25-hydroxy vitamin $D_3$ antibodies. Linkage of the hapten to the carrier protein is preferably by the $C_{1-10}$ alkylene chain.

The compound of formula (I) can be prepared by reacting 25-hydroxy vitamin $D_3$ with an amino acid of

formula (IV):

$HOOC-R_1-NH_2$

wherein $R_1$ is as defined above, the amino acid having a protected amino group, to obtain a 25-hydroxy vitamin $D_3$ derivative of formula (II):

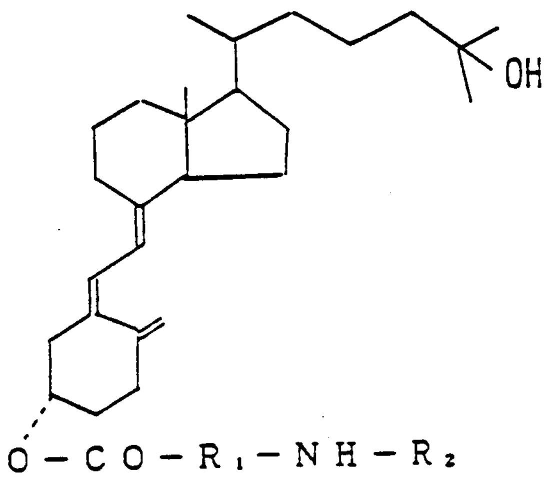

and removing the protecting group therefrom.

The present invention also provides a process for preparing a 25-hydroxy vitamin $D_3$ amino acid derivative of formula (I) as defined above wherein a compound of formula (II):

(II):

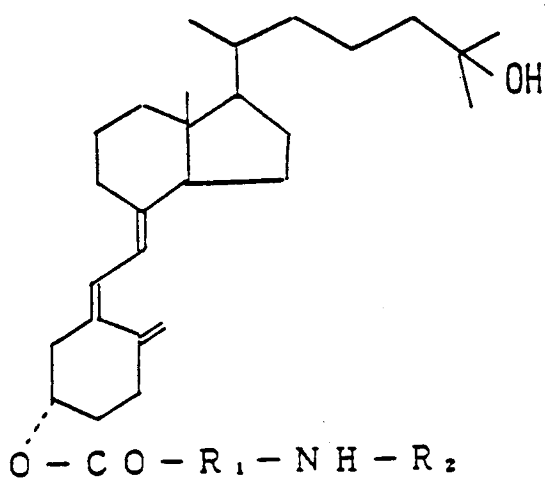

wherein $R_1$ is as defined above and $R_2$ is an amino-protecting group, is deprotected in the presence of a base and in an inert solvent.

Examples of the number of carbon atoms in the $R_1$ group, and compounds from which they may be obtained, are: n = 1; glycine, n = 2; β-alanine, n = 3; γ-amino-butyric acid and β-amino-iso-butyric acid, n = 4; δ-amino valeric acid, n = 5; ε-amino-n-caproic acid, n = 6; 7-amino heptanoic acid, and n = 10; 11-amino undecanoic acid. The carbon chain in $R_1$ is preferably straight, and also preferably contains from 2 to 6 carbon atoms. δ-amino-levulinic acid, glycylglycine or δ-amino acid, ie D and/or L-lysine can also be used. An amino acid having a side chain hydroxyl, for example 4-amino-3-hydroxybutyric acid can be used if the hydroxyl group is protected. Thus the alkylene group represented by $R_1$ may be unsubstituted or

substituted, for example by at least one amino, hydroxy or oxo group.

Suitable amino protecting groups are those which can easily be removed in mild conditions such as weakly basic conditions due to the possible instability of vitamin D under more severe removal conditions. Examples are 9-fluorenyl methyloxycarbonyl (hereinafter designated as Fmoc), 9-(2-sulfo)-fluorenyl methyloxycarbonyl, 1,1-dimethyl-2-cyanoethyloxycarbonyl and 5-benzisoxazolyl methyloxycarbonyl groups, most preferably a 9-fluorenyl methyloxycarbonyl group due to its ease in use. An amino-protected compound can be made by known methods by reacting an activated derivative, for example an activated ester of an Fmoc containing compound such as a 9-fluorenylmethyl-succinimidylcarbonate or 9-fluorenyl methylchloroformate derivative, with an amino acid [L.A. Carpino and G.Y. Ham. J. Org. Chem., 37: 3404 (1972)].

The 25-hydroxy vitamin $D_3$ amino acid derivative of formula (II) can be obtained, for example using Fmoc-amino acid as an amino protected amino acid, by reacting one equivalent of 25-hydroxy vitamin $D_3$ with one equivalent of Fmoc-amino acid or its acid anhydride, acid halide or activated ester. Preferably, one equivalent of Fmoc-amino acid is reacted with one equivalent of a mixed anhydride with another acid under an inert gas and in the presence of a base in an anhydrous solvent. A reason for reacting the mixed anhydride with 25-hydroxy vitamin $D_3$ is to prohibit formation of a by-product, 25-hydroxy vitamin $D_3$ having a Fmoc-amino acid group attached to the hydroxy group at postion-25.

Examples of other acids in a mixed anhydride are valeric acid, pivalic acid and isobutylchloroformate; pivalic acid is preferred. Examples of anhydrous solvents are anhydrous organic solvents such as tetrahydrofuran or dioxane. Examples of bases are dimethylamino pyridine (DMAP) and piperidinopyridine (PPY), which are preferred for esterification of a secondary alcohol or tertiary alcohol with steric hindrance. A preferred inert gas is argon or nitrogen. The reaction preferably proceeds at 0-20 °C for 1-3 hours. The thus obtained 25-hydroxy vitamin $D_3$ derivative of formula (II) can be purified, if necessary, by a purification method such as column chromatography or thin layer chromatography (TLC).

The 25-hydroxy vitamin $D_3$ derivative of formula (II) is subjected to de-protection of the amino group, ie removal of Fmoc, in the presence of a base in an inert solvent to produce a 25-hydroxy vitamin $D_3$ derivative of formula (I). Examples of bases are piperidine, morpholine and ethanolamine; morpholine is most preferred. Examples of inert solvents are ethanol and methanol, preferably anhydrous ethanol or anhydrous methanol. The reaction proceeds under an inert gas in the dark at 0 - 20 °C for 1 - 3 hours.

The thus obtained 25-hydroxy vitamin $D_3$ derivative of formula [I ] can he purified by column chromatography or TLC.

In a radioisotope iodine labelled 25-hydroxy vitamin $D_3$ derivative of formula [III ] , $R_1$ has the same meaning as in formula [I ] and $R_3$ is a group containing an iodine radioisotope. Examples of an iodine radioisotope are $^{125}I$ and $^{131}I$; the relatively long half-life isotope are $^{125}I$ is preferred. Examples of such groups are 3-(4-hydroxy-3-iodo [$^{125}I$] phenyl)-propionyl, 3-(3,5-diiodo [$^{125}I$] -4-hydroxyphenyl) propionyl, 2-(4-hydroxy-3-iodo [$^{125}I$] phenyl) acetyl, 2- (3,5-di-iodo [$^{125}I$] -4-hydroxyphenyl) acetyl, 2-iodo [$^{125}I$] acetyl, 4-iodo [$^{125}I$] benzoxymethyl carbonyl and an N-substituted-3-iodo [$^{125}I$] tyrosin residue. 3-(4-hydroxy-3-iodo [$^{125}I$] phenyl) propionyl and 3-(3,5-diiodo [$^{125}I$] -4-hydroxyphenyl) propionyl are preferred.

In the production of an iodine radioisotope labelled 25-hydroxy vitamin $D_3$ derivative, the 25-hydroxy vitamin $D_3$ amino acid derivative of formula [I ] is labelled with an iodine radioisotope by an indirect labelling method by means of a Bolton-Hunter reagent to obtain in the iodine radioisotope labelled 25-hydroxy vitamin $D_3$ derivative. An indirect labelling method is a method for producing an iodine radioisotope labelled 25-hydroxy vitamin $D_3$ derivative of formula [III ] by reacting a reactive derivative of formula [IV] as defined below, which has an iodine radioisotope labelled group, with a 25-hydroxy vitamin $D_3$ amino acid derivative of formula [I ].

The reactive derivative of formula [IV] has the formula:

$R_3$ -X [IV]

wherein $R_3$ is as defined in above and X is a succinimidyl-N-oxy, phthalimidyl-N-oxy, 5-norbornene-2,3-dicarboximidyl-N-oxy or maleimidyl-N-oxy group. N-succinimidyl-3-(4-hydroxy-3-iodo [$^{125}I$] phenyl) propionate, for which $R_3$ is 3-(4-hydroxy-3-iodo [$^{125}I$] phenyl) propionyl and X is succinylimidyl-N-oxy in the compound of formula (IV) , is a commercially available [$^{125}I$] Bolon-Hunter reagent. An indirect labelling method can be carried out on a 25-hydroxy vitamin $D_3$ amino acid derivative of formula [I ] using said Bolton-Hunter reagent by, for example, reacting several p moles to several m moles of iodine [$^{125}I$] radioisotope Bolton-Hunter reagent with a 500 $\sim$ 2000 excess amount, preferably a 1000 excess amount, of 25-hydroxy vitamin $D_3$ amino acid derivative, at 0 - 30 °C for 12 - 72 hours. In order to increase an effect on a RIA-or CPBA-method, the produced radioisotope labelled 25-hydroxy vitamin $D_3$ derivative of formula [III ]

is preferably purified by TLC or HPLC. The thus obtained $^{125}$ I-labelled 25-hydroxy vitamin $D_3$ derivative is stable at -20°C in ethanol for more than two months of the half-life of $^{125}$I, and can be used in a radio immunoassay.

The stock-temperature is preferably as low as possible, ie 5 °C ∿ -20°C, preferably below -20°C. The derivative may be stored in alcohol or ether under an inert gas.

Anti 25-hydroxy vitamin $D_3$ antibody can be prepared by inoculating a conjugate of a hapten, i.e a 25-hydroxy vitamin $D_3$ amino acid derivative of formula [I ], and a carrier protein into an animal. Examples of a carrier protein, which is essential for obtaining an immunogenic antigen for the hapten, are simple proteins, polypeptides and complex proteins such as a glycoprotein. Examples of a simple protein are bovine serum albumin (BSA), human serum albumin or human serum globurin. An example of a polypeptide is polylysine. An example of a glycoprotein is mucoprotein. A simple protein is preferred, especially bovine serum albumin and human serum albumin.

The 25-hydroxy vitamin $D_3$ amino acid derivative of formula [I ] and a carrier protein are joined by a covalent bond in the presence of a condensation reagent or crosslinking reagent. Examples of condensation reagents and crosslinking reagents are dicyclohexylcarbodiimide (DCC), acid anhydride and glutaraldehyde; DCC is most preferred. The conjugation ratio of the 25-hydroxy vitamin $D_3$ amino acid derivative to the carrier protein can be, due to a decreasing titer of antibody if one is in excess, such that 10 ∿ 40 molecules of 25-hydroxy vitamin $D_3$ amino acid derivative are present in one molecule of carrier protein.

The thus prepared conjugate for antibody production is inoculated into an animal to produce an antibody. Inoculation can be made by parenteral administration such as subcutaneous or intracutaneous injection. A conjugate antigen of the above 25-hydroxy vitamin $D_3$ amino acid derivative-carrier protein is dissolved in a buffer solution or physiological saline, together with an equi-amount of complete Freund's adjuvant (C.F.A.), the mixture is emulsified completely, and inoculated subcutaneously or intracutaneously into a homeothermal animal, for about 10 times every 1∿ 3 weeks for immunization. Alternatively the conjugated antigen can be directly inoculated into a spleen. During an the immunization period, serum antibody titer is measured at various times and at maximum titer whole blood sample is collected and allowed to stand for coagulation. The coagulated sample is centrifugally separated to obtain antiserum containing the anti 25-hydroxy vitamin $D_3$ antibody.

The nature of the homeothermal animal is not limited; it can be an animal which has antibody production activity. It is preferred to obtain a large amount of antibodies from sheep or bovine animals. In general a rabbit or rat is used.

Isolation of anti 25-hydroxy vitamin $D_3$ antibody from antiserum can be by a conventional method for antibody purification. For example, ammonium sulfate fractionated antiserum is treated by ion-exchange chromatography or gel-filtration.

Another method of production of the antibody is by inoculating spleen cells, which can produce the desired antibody of the animal, with the conjugate antigen of 25-hydroxy vitamin $D_3$ amino acid derivative-carrier-protein, and fusing the cells with established myeloma cells. The thus obtained hybridoma is cultured and the monoclonal antibody which is produced by the hybridoma is used.

For example, an emulsion, which is prepared by mixing a conjugate antigen of 25-hydroxy vitamin $D_3$ amino acid derivative-carrier protein dissolved in buffer solution or physiological saline and an equal amount of C.F.A., is inoculated subcutaneously in a mouse, for example of strain Balb/c for sensitization. Cell-fusion is performed 3 ∿ 5 days after final sensitization. 3 ∿ 5 days after final sensitization, spleen cells which produce anti 25-hyroxy vitamin $D_3$ antibodies are collected and fused with established myeloma cells which can be cultured for a long term. A long-term culturable established cell may be a cell which can be cultured and grown for a long term in vitro or in vivo, which can produce immunoglobulin or its related proteins; in general well grown myeloma cells are used. Preferred myeloma cells are cell-lines P3-NSI/1-Ag4-1, P3-X63-Ag8U1, SP2/U-Ag14 and MPC11-45.6.TG.1.7. In the present invention, P3-X63-Ag8U1 is preferred. The cells can be cultured in a conventional cell culture medium. Culture can, for example, be performed in a medium of 10 % FCS added RPM1 1640 (Tradename, Flow Laboratory), which is added with glutamine, pyruvic acid, penicillin and streptomycin. For stock culture, S-azaguanine is added to the above medium. 1 ∿ 3 X $10^8$ myeloma cells, are used for cell-fusion. Spleen cells can be prepared by cutting a mouse spleen and crushing it on a mesh to prepare a spleen cell suspension. Washed cells, generally 1∿ 3 X $10^8$ cells, are fused with myeloma cells by mixing each cell. The ratio of cells which are mixed is experimentially myeloma cells: spleen cells 1 : 3 ∿ 10. Cell-fusion is achived in a medium for hybridomas. A conventional cell-fusion method using a promotor such as Sendai-virus or polyethylene glycol (PEG) is preferred. PEG is most preferred in the present invention.

Fused cells are inoculated into the medium for hybridomas, incubated, then selected by incubating in HAT medium. HAT medium is a medium for hybridomas with added hypoxanthine, aminopterin and

thymidine. Since more than 2 hybridomas are generally grown in the well of a cell-separation plate, more than two kind of antibodies are possibly produced or no-antibody producing cells may be contaminated. In order to obtain cells having the same properties, each clone should be separated. For the cloning, a limiting dilution culture or soft agar culture is used. In this invention, limiting dilution culture is preferred.

The thus obtained hybridoma secreting anti 25-hydroxy vitamin $D_3$ antibody with high titer can be stored by lyophilization at an early stage. Lyophilization can be made by a conventional method, namely a cell suspension in a small tube or ampule is frozen in a -80 °C freezer and stored in liquid nitrogen. Another example of hybridoma production is that the above hybridoma is inoculated intraperitoneally in pristan (2,6,10,14-tetramethylpentadecane, Aldrich Chemicals) treated mice, and after about 10 days ascites are collected. A further method is that the hybridoma is incubated in bovine fetal serum added RPMI medium or in Darbecco modified Eagle medium. The antibody thus obtained can be purified by a conventional method. For example the antibody is fractionated with ammonium sulfate and treated by ion-exchange chromatography, gel-filtration and affinity chromatography to fractionate IgG. Then purified anti 25-hydroxy vitamin $D_3$ monoclonal antibody can be obtained.

Furthermore, anti 25-hydroxy vitamin $D_3$ monoclonal antibody producing cells can be inoculated in and grown in an animal having an identical histocompatibility antigen or in a nude mouse as a tumor, grown cells collected and monoclonal antibody purified therefrom.

In an assay of 25-hydroxy vitamin $D_3$, anti 25-hydroxy vitamin $D_3$ polyclonal antibody or anti 25-hydroxy vitamin $D_3$ monoclonal antibody (hereinafter sometimes both referred to as anti 25-hydroxy vitamin $D_3$ antibody) can be used in a soluble state or in an immobilized state. Insoluble carrier and anti 25-hydroxy vitamin $D_3$ antibodies are bound using a polyfunctional reagent and the immobilized antibody has an antibody titer against 25-hydroxy vitamin $D_3$. Examples of polyfunctional reagents are compounds having more than two groups which can react with functional groups such as amino, hydroxyl , carboxyl and thiol, such as aldehyes such as succinaldehyde, glutaradehyde or adipoaldehyde, dicarboxylate such as malonicacid, succiniacid glutaraiccid or adipicacid or its reactive derivative, diisocyanates such as hexamethylene diisocyanate or 2,4-toluenediisocyanate, diisothiocyanate such as hexamethylene diisothiocyanate, maleimide carboxylates such as maleimide benzonate or maleimide phenylacetate or its functional derivative, dimaleimides such as N,N-ethylene-bis-maleimide or N,N'-O-phenylene dimaleimide, bisdiazobenzidine, diethylmalonimidate, dimethyladipinimidate or N,N'-polymethylene-bisiodo acetamide and thiocarboxylates such as 3-(2'benzothiozolyl-dithio) propionate or 3- (2'-pyridyldithio) propionate or its functional derivative. The polyfunctional reagent can be selected by considering the bonding of a functional group such as amino, carboxyl, hydroxyl or thiol in an anti 25-hydroxy vitamin $D_3$ antibody. An immobilized carrier is a carrier having a reactive group which does not bind with a group for bonding with an antibody in a polyfunctional group. Examples of immobilized carriers are insoluble proteins such as albumin or gelatin, epichlorhydrin treated insoluble polysacharides such as agarose, cellulose or dextrin, insoluble polymers or copolymers of acrylonitrile, acrylic acid, aclylate ester, metacrylic acid, metacrylate ester, vinylalcohol, vinylacetate, styrene, aminostyrene, chlorstyrene, maleic acid or fumaric acid, which is treated with bromocyanate and introduced with spacer corresponding to amino group introduction, and insoluble inorganic carriers introduced with a funtional group such as an amino group into an organic compound of for example, silicon or aluminium. An immobilized carrier may also be a carrier which can bind an anti 25-hydroxy vitamin $D_3$ antibody by physical adsorption.

An immobilized carrier is preferably in the form of particles which can easily isolated by filtration, for example beads having diameter of more than 1 mm, preferably more than 5 mm, or in a spindle form which corresponds to the bottom shape of an antigen-antibody reaction tube.

Introduction of a reactive group into anti 25-hydroxy vitamin $D_3$ antibody using a spacer introducing reagent can be made by introducing an additional functional group such as aldehyde, carboxyl, amino or thiol, and reacting with one or more spacer introducing reagents, for example dialdehydes such as succinaldehyde, glutaraldehyde or adipoaldehyde, reactive derivatives such as acid chloride, succinimide ester or p-nitrophenyl ester of ω-amino butyric acid or ω-amino glutamic acid, reactive derivatives of dicarboxylic acids such as malonic acid, succinic acid, glutaric acid or adipic acid, diamines such as hexamethylene diamine or decamethylene diamine, reactive derivatives of 3- (2' -pyridyl-dithio) propionic acid or 3-(2'-benzothiazolyl-dithio) propionic acid, S-acetylmercap to succinic anhydride or thiols such as 2-aminoethanethiol.

Anti 25-hydroxy vitamin $D_3$ antibody is condensed directly or through a polyfunctional reagent with a reactive group in the immobilized carrier. The Condensation reaction proceeds generally at 0 - 40 °C in a pH 6.0~ 8.5 buffer solution or organic solvent or mixture thereof. Furthermore, a second antibody which is obtained by immunizing large mammals inoculated with an immunoglobulin fraction in serum, which is used for antibody production of 25-hydroxy vitamin $D_3$, is immobilized and anti 25-hydroxy vitamin $D_3$ antibody is

bound thereto by an antigen-antibody reaction to prepare the immobilized antibody.

The thus obtained immobilized antibody is washed and stored.

In an assay of 25-hydroxy vitamin $D_3$ in a specimen using anti 25-hydroxy vitamin $D_3$ antibody in a liquid soluble phase, a fixed amount of iodine radioisotope labelled 25-hydroxy vitamin $D_3$ derivative of formula [III ] is previously added to a specimen containing 25-hydroxy vitamin $D_3$, then an optimum amount of anti 25-hydroxy vitamin $D_3$ antibody is added to form an antigen antibody combining reaction product.

The thus formed labelled 25-hydroxy vitamin $D_3$derivative-anti 25-hydroxy vitamin $D_3$ antibody binding complex, 25-hydroxy vitamin $D_3$-antibody binding complex, and free labelled 25-hydroxy vitamin $D_3$ derivative are separated by using a specific antibody for anti 25-hydroxy vitamin $D_3$ antibody. The said specific antibody is called the second antibody. The second antibody can be obtained, for example by inoculating a normal immunoglobulin fraction in the serum of an animal which is used for antibody production of 25-hydroxy vitamin $D_3$, as an antigen to immunize, then isolating the antibody from the thus obtained anti-serum. The second antibody can be purified if necessary by a known method, or it can be preferably be used in the state of an anti-serum.

An assay method of 25-hydroxy vitamin $D_3$ in a specimen using anti 25-hydroxy vitamin $D_3$ and iodine radioisotope labelled 25-hydroxy vitamin $D_3$ derivative is illustrated as follows:

25-hydroxy vitamin $D_3$ in a specimen such as a known serum, is extracted from the serum specimen. A sample, which is a mixture of serum and an equal amount of added solvent, is stirred, allowed to stand and centrifuged. Separated supernatant solution is treated by column chromatography and a fraction containing 25-hydroxy vitamin $D_3$ is collected. The sample is preferably purified by HPLC.

The 25-hydroxy vitamin $D_3$ fraction can be checked by previously added tritium [$^3$H] labelled 25-hydroxy vitamin $D_3$. The 25-hydroxy vitamin $D_3$ fraction is dried in vacuo, the vacuum replaced by argon gas, and the fraction dissolved in ethanol to prepare a specimen. A fixed amount of iodine radioisotope labelled 25-hydroxy vitamin $D_3$ derivative is added to a specimen, and the most suitable amount of anti 25-hydroxy vitamin $D_3$ antibody is added thereto. The mixture is incubated in a medium for antigen-antibody such as a phosphate buffer or veronal buffer at 4 - 5° C for about 15 - 72 hours to allow a competitive reaction of iodine radioisotope labelled hapten and non-labeled hapten to antibody to proceed. The thus formed antigen antibody binding complex, namely a bound form of iodine radioisotope labelled 25-hydroxy vitamin $D_3$ derivative-anti 25-hydroxy vitamin $D_3$ antibody (B) and an unreacted free form of iodine radioisotope labelled 25-hydroxy vitamin $D_3$ derivative (F), are separated b a dextran-charcoal (DC) method with filtration or centrifugation at 3,000 r.p.m. for 15 minutes. On B-F separation, the ratioactivity in B or F is measured.

The amount of 25-hydroxy vitamin $D_{3,}$(H) in a specimen is calculated by measuring the radioactivity and calculating B/(B + F) or B/F. When the amount of H is increased the radioactivity of B is decreased and that of F is increased. Therefore the unknown amount H can be obtained by measuring the radioactivity of B and F from a previously set up standard curve of a known amount.

On the B-F separation, when a double antibody technique is applied in a soluble state antibody, a second antibody, preferably a second antibody containing antiserum and if necessary normal serum of the same kind of animal used for the anti 25-hydroxy vitamin $D_3$ antibody production, are added after competitive reaction, and incubated for 1 ~ 12 hours. Thereafter the formed binding complex is precipitated by centrifugation at 3,000 r.p.m. for 10 ~ 30 minutes to separate precipitate (B) and supernatant (F), and then the radioactivity of B or F is measured.

According to the present assay method, a standard curve on 2 pg/Test~ 256 pg/Test can be prepared, and a rapid reaction time of 16 hours at 5° C or 1 hour at 37° C can be achieved. Further operation after the reaction is quite simple. Moreover dilution and recovery tests using a specimen show a linearity with high precision. The correlation coefficient $\tau$ between the [$^{125}$I] RIA method of the present invention and a known [$^3$H] CPBA method on an assay of 25-hydroxy vitamin $D_3$ in human serum is 0.980 ($y = 0.928X + 1.90$, $n = 18$).

When an antibody is replaced by known DBP, it can be reacted with an iodine [$^{125}$I] radioisotope labelled 25-hydroxyvitamin $D_3$ derivative and a standard curve between 1 ~ 32 pg/Test can be prepared. The DBP can be obtained from the serum of a chicken, rat, mouse, rabbit, goat, sheep, bovine animal or human.

The following Examples further illustrate the present invention.

<u>Referential Example 1:</u>

Extraction and purification of 25-hydroxy vitamin $D_3$ from serum :

A mixture of 0.5mℓ serum and 0.5mℓ acetonitrile was stirred by a BORTEX mixer and then allowed to stand for 30 minutes. 0.8 mℓ supernatant solution obtained by centrifugation at 3,000 r.p.m. for 10 min, was charged on a Sep-pack C - 18 cartridge column (tradename, MILLIPORE, Waters Corp.) which was activated with ethanol, equilibrated with 50% acetonitrile and eluted with hydrated acetonitrile. The column was then washed with 4mℓ 50% acetonitrile then eluted with 4mℓ 64% acetonitrile (a fraction of 1 α, 25-dihydroxyvitamin $D_3$), and eluted with 4mℓ 73% acetonitrile (a fraction of 25-hydroxy vitamin $D_3$ and 24, 25-dihydroxy-vitamin $D_3$). The fraction eluted with 73 % acetonitrile was dried in vacuo, and packed with argon gas to obtain a fraction containing 25-hydroxy vitamin $D_3$. The thus obtained crude fraction was dissolved in a 200 μℓ mixture of n-hexane-isopropanol (9 : 1) and purified by HPLC on a Zorbax-SIL (Dupont Inc.) 0.46 X 25 cm column. The above fractions of 25-hydroxy vitamin $D_3$ were checked by previously added tritium [$^3$H] labelled 25-hydroxy vitamin $D_3$ [ 26, 27-methyl-$^3$H ] . The fraction of 25-hydroxy vitamin $D_3$ (Rt = 4 ∼ 5 min) was dried in vacuo and packed with argon gas.

The said fractions were dissolved in 2 mℓ ethanol and 20μℓ thereof was used for an assay.
Recovery of 25-hydroxy vitamin $D_3$ is 94.6± 2.4 % (n = 18).

Referential Example 2:

3- (N-fluorenyl methyloxy carbonyl) amino propionic acid :

τ -amino-n-propionic acid (178 mg, 2 mM) was added to N-succinimidyl-9-fluorenylmethyloxycarboxylate (674 mg, 2 mM) dissolved in a mixture of tetrahydrofuran ( THF )-dimethylformamide (DMF)-$H_2$0 (1 : 2 : 2) 25 mℓ and reacted at room temperature overnight. The reaction solvent was distilled off in vacuo and the residue was charged on a column of silica-gel (Wako-gel C-200, 75 g) and separated and purified by eluting with CHCℓ$_3$ : methanol = 9 : 1 to obtain 538.8 mg of a compound ( yield : 86.5 % ).

NMR. δ (DMSO-$d_6$)
2.50 - 2.60 ( 2H, t, -$CH_2$-Co-)
3.28 - 3.35 ( 2H, m, -$CH_2$-N-)
4.33 - 4.44 ( 3H, m, Fmoc )
7.35 - 8.06 ( 8H, m, Fmoc )

Example 1 :

25-hydroxy vitamin $D_3$-3β-0-3 (3'-( N-9-fluorenyl methyloxy carbonyl) aminopropionate):

Pivaloyl chloride (7.38μℓ d = 0.979, 0.06 mM) and dimethylamino pyridine (DMAP, 7.32 mg, 0.06 mM) were added to 3-( N-9-fluorenyl methyloxy carbonyl) amino propionic acid (18.7 mg, 0.06 mM) dissolved in 3mℓ dry THF, and reacted at -15°C for 15 minutes under an argon atomsphare in the dark. 1 ml of a THF solution of 25-hydroxy vitamin $D_3$ (24.0 mg, 0.06 mM) was added thereto and reacted at 0 °C for 1 hour and at room temperature for 1 hour; 25-hydroxy vitamin $D_3$ disappeared. 0.5 mℓ methanol was added to the mixture to stop the reaction, then distilled off in vacuo. The residue was purified by preparative TLC [Art. 5717, Merck, 20x 20 cm, developer : ethylacetate-hexane ( 1 : 2 ) ] to obtain 30.1 mg of a compound ( yield 72.4 %).

NMR. δ d (CDCℓ$_3$ ) p p m
0.537 ( 3H, s, $CH_3$-18 )
3.30 - 3.60 ( 2H, m, -$CH_2$-N- )
4.10 - 4.50 ( 3H, m, Fmoc )
4.80 - 5.60 ( 4H, m, H-19E, H- 3α, H-19Z, -NH )
5.94 6.29 ( 2H, m, H-7, H-6)
7.20 - 7.81 ( 8H, m, Fmoc )

UV. $\lambda^{EtOH}_{max}$ nm 300.2, 266.4, 214.4

Example 2 :

25-hydroxy vitamin $D_3$- 3 $\beta$-0-(3-aminopropionate):

25-hydroxy vitamin $D_3$-3$\beta$-0- [3-( N-9'-fluorenyl methyloxy carbonyl) aminopropionate ] ( 30.1 mg, 0.043 mM) obtained in Example 1 was dissolved in 1 mℓ ethanol. 10 m ℓ morpholine was added thereto and stirred at room temperature for 1 hour under an argon atomosphere in the dark to complete the reaction. The reaction mixture was concentrated in vacuo and purified by preparative TLC [Art. 5717, Merck, 10X 20 cm, developer : ethyl acetate-MeOH (1 : 4 ) ] to obtain 8.3 mg of a compound ( yield 40.6 %).

Ninhydrin colorization : positive

NMR. $\delta$ (CDC ℓ$_3$ ) p p m
0.54 ( 3H, s, $CH_3$-18 )
2.36 - 2.52 ( 2H, m, -C0-$CH_2$-)
2.90 - 3.05 ( 2H, m, -$CH_2$-N- )
3.50 - 4.00 ( 2H, b, -$NH_2$ )
4.86 - 5.08 ( 3H, m, H-19E, H- 3$\alpha$,H-19Z)
5.90 - 6.30 ( 2H, m, H-7, H-6)

$$UV.\ \lambda_{max}^{EtOH}\ nm\quad 264.5$$

Example 3 :

25-hydroxy vitamin $D_3$- 3 $\beta$-0 - [3 -(BSA-amino) propionate ] antigen and : 25-hydroxy vitamin $D_3$antiserum :

(1) Preparation of 25-hydroxy vitamin $D_3$- 3 $\beta$-0- [3-(BSA-amino) propionate ] antigen.

25-hydroxy vitamin $D_3$ -3 $\beta$ -0- [3 -aminopropionate (18.1 mg, 38.43 X 10 $^{-3}$ mM ) obtained in Example 2 was dissolved in 1 m ℓ THF, and was added to BSA (M.W. 65,000, 50 mg, 1/50 X 38.43 X $10^{-3}$ mM) dissolved in Tris-buffer (0.1 M, pH 8.6 ) at 0°C with 1-ethyl-3-(-3dimethylaminopropyl)-carbodiimide hydrochloride (9.6 mg,1.3 X 38.43 X $10^{-3}$ mM). 10 m ℓ chloroform was added 4 times to the reaction mixture and unreacted 25-hydroxy vitamin $D_3$- 3$\beta$-0- (3 - aminopropionate ester) was removed. The aqueous layer was freeze dried to obtain 45 mg lyophilized product which was a complex of 22 molecules of 25-hydroxy vitamin $D_3$-3$\beta$ -0- (3 -amino propionate ester) bound through the amino groups at position-3 thereof on one molecule of BSA.

(2) Preparation of 25-hydroxy vitamin $D_3$ antiserum :

Lyophilized 25-hydroxy vitamin $D_3$- 3 $\beta$-0- [3 -(BSA-amino) propionate ] (antigen) obtained in the above (1) was dissoleved in Tris buffer (0.1 M, pH 8.6). An equal amount of C.F.A. was added thereto and mixed to emulsify to prepare 1 $\mu$ g ~ 200 $\mu$g/m ℓ antigen. The emulsion was inoculated subcutaneously 10 times for every 2 weeks, 50$\tau$ - 500 $\tau$ /head in a rabbit. During immunization, a blood sample titer collected every 10 days was measured and at maximum antibody titer whole blood was collected. The blood sample was allowed to stand at room temperature for 60 minutes to coagulate and centrifuged at 3,000 r.p.m. for 10 min to obtain antiserum containing anti 25-hydroxy vitamin $D_3$ antibody, which was fractionated with ammonium sulfate to collect the IgG fraction.

(3) Preparation of anti 25-hydroxy vitamin $D_3$ monoclonal antibody :

Lyophilized 25-hydroxy vitamin $D_3$-3$\beta$-0- [3 -(BSA-amino) propionate ] (antigen) obtained in the above (1) dissolved in phosphate buffer, pH 7.2, 50 $\tau$ together with C.F.A. was inoculated subcutaneously in Balb/c mice, female, of 4 weeks age. After 1 week 50 $\tau$ thereof was subcutaneously inoculated and after 2 weeks 50 $\tau$ of antigen was intraperitoneally inoculated. On 3 days after the final inoculation, the spleen was finely cut and crushed on a mesh to prepare a spleen cell suspension. Cell fusion was performed using mouse myeloma cells P3-X-3-Ag8U1 by a conventional method. 30 % PEG (M.W. 1,000) aqueous solution was incubated at 37 °C. The spleen cells and myeloma cells (total 4 x $10^7$ cells, 5 : 1) were suspended in RPMI medium (5 m ℓ) and both cells were gently mixed and centrifuged at 1.000 r.p.m. for 10 minutes. The supernatant was then vacuum filtered. The test tube was weaky shaken to mix the cell pellets and 1.0 m ℓ PEG solution was slowly added and gently stirred. The mixture was incubated at 37 °C with gentle shaking, then the fusion reaction was stopped by adding slowly conventional medium (10 m ℓ) and again cells curve suspended. The suspension was centrifuged at

1.000 r.p.m. for 5 minutes. The precipitated cell pellet was dispersed by gently shaking and was slowly suspended in HAT medium ( 5 m ℓ) and was transferred into HAT-medium (1 m ℓ) in a vessel. The cells were observed microscopically. 200 ml of the cell suspension was pipetted into each well of a 96 well plate and grown in the $CO_2$-incubator. The fused cells were selected in HAT medium for cloning by the limiting dilution technique. The obtained clone suspension was grown in a peritoneal of pristan treated Balb/c mouse. The IgG fraction was collected by a conventional method from ascites and serum and purified by affinity chromatography using protein A bound Sepharose CL-4B. The thus obtained IgG subclass was IgG 1, which was used as a monoclonal antibody.

Example 4 :

Preparation of monoiodine [$^{125}$I] radioisotope labelled 25-hydroxy vitamin $D_3$ derivative ; 25-hydroxy vitamin $D_3$- 3β-O - {3- [N-3-(4-hydroxy-3-iodo [$^{125}$I] phenyl) propionyl ] amino propionate} :

Iodine [$^{125}$I] Bolton-Hunter reagent [NEN, NEX-120-10, 2200 Ci/mM, Total 0.33 mCi/100μ ℓ Benzene solution ] (100 pM/66.6 μ ℓ/220μCi) and DMAP (1 nM/1μ ℓ THF) were added to a THF solution (130 μ ℓ) of 25-hydroxy vitamin $D_3$-3β-O-(3-aminopropionate ) (100 nM/127.8μ ℓ THF) obtained in Example 2, and reacted at room temperature for 24 hours under an argon atomosphere in the dark. The reaction mixture was purified by HPLC (Zorbax-SIL, 4.6 mm X 25 cm column, 20 % isopropanol-n-hexane, flow 1 m ℓ /min). The compound hereinabove has Rt = 8.50 - 9.50 min and recovery 20.0 %. (Iodine [$^{125}$I] Bolton-Hunter reagent : Rt = 14.0 - 16.0 min). Radioisotope monoiodine labelled 25-hydroxy vitamin $D_3$ was identified with synthesized non-radio isotope monoiodine labelled 25-hydroxy vitamin $D_3$ by HPLC.

Example 5 :

Preparation of diiodine [$^{125}$I] radioisotope labelled 25-hydroxy vitamin $D_3$ derivative ; 25-hydroxy vitamin $D_3$ -3β-0 - {3- (N-3,5-diiodo-4-hydroxy [$^{125}$ I ] phenyl)propionyl ] amino propionate) :

Iodine [$^{125}$I] Bolton-Hunter reagent [NEN, NEX-120H-10 , 4400 Ci/mM, Total 0.33 mCi/100μ ℓ Benzene solution ] (25 pM/33.3 μ ℓ/110μCi) and DMAP (1 nM/1 μ ℓ THF) were added to a THF solution (120 μ ℓ) of 25-hydroxy vitamin $D_3$-3β-0-(3 - aminopropionate ) (25 nM/6.952 μ ℓ THF) obtained in Example 2, and reacted at room temperature for 20 hours under an argon atomosphere in the dark. The reaction mixture was purified by HPLC (Zorbax-SIL, 4.6 mm , X 25 cm column, 20 % isopropanol-n-hexane, flow 1 mℓ/min). The compound hereinabove has Rt = 8.90 - 10.10 min and recovery 9.9 %. (Iodine [$^{125}$I] Bolton-Hunter reagent : Rt = 14.0 - 16.0 min.). Di-iodine radioisotope labelled 25-hydroxy vitamin $D_3$ was identified with synthesized di-iodine non-radioisotope labelled 25-hydroxy vitamin $D_3$ by HPLC.

Example 6 :

Evaluation of iodine [$^{125}$I] radioisotope labelled 25-hydroxy vitamin $D_3$ derivative :

(1) Preparation of standard curve using radioimmuno assay of 25-hydroxy vitamin $D_3$ :

Standard ethanol solution of 25-hydroxy vitamin $D_3$ 256 pg/20 μ ℓ was diluted in a series manner by double in double dilution to prepare 128 pg/20μ ℓ, 64 pg/μ ℓ, 32 pg/20 μ ℓ,16 pg/20μ ℓ, 8 pg/20 μ ℓ, 4 pg/20 μ ℓ and 2pg/20 μ ℓ solutions.

Samples (20μ ℓ) of each concentration hereinabove were pipetted into 3 tubes. 20 μl ethanol solution of iodine [$^{125}$I] radioisotope labelled 25-hydroxy vitamin $D_3$ derivative which contained antioxidant tocopherol was pipetted into each tube (20 μ ℓ ca. 13,000 cpm).

1 ml anti 25-hydroxy vitamin $D_3$ rabbit serum diluted with Tris buffer (approx. 15,000 dilution) pH 8.6 was added to each tube. Each mixture in the tubes was stirred by a BORTEX mixer and allowed to stand at 5 °C overnight (20 - 24 hours). Glycine buffer, pH 8.6 suspended with a mixture of dextran T70 and active charcoal (1 : 10), (each 300 μ ℓ) was added into the reaction tubes, stirred by a Bordex mixer and incubated at 5 °C for 1 hour. B-F separation was made by centrifugation at 3,000 r.p.m. for 10 minutes, and 1 m ℓ of each supernatant solution was measured by an autowell τ-counter for 1 min. A binding ratio is calculated by the following equation:

$$\text{Binding ratio} = \frac{(B) - (BL)}{(Bo) - (BL)} \times 100$$

wherein (B) is the count No. in each tube, (BL) is the count No. using 25-hydroxy vitamin $D_3$ (1,000 pg/20$\mu$ ℓ) in place of $^{125}$I labelled 25-hydroxy vitamin $D_3$ derivative, and (Bo) is the count No. measured using 25-hydroxy vitamin $D_3$ at zero concentration. Fig.1 shows the standard curve of [$^{125}$I] labelled 25-hydroxy vitamin $D_3$ derivative.

(2) Comparison with tritium [$^3$H] labelled and [$^{125}$I] labelled 25-hydroxy vitamin $D_3$ derivative :

A standard curve was prepared by using tritium [$^3$H] labelled 25-hydroxy vitamin $D_3$ derivative (26, 27-methyl-$^3$H) in the same manner as in the [$^{125}$I] labelled derivative in (1) hereinabove.

The result is shown in Fig. 2. In Fig. 2, - ●- ●-: [$^{125}$I] labelled and - ▲- ▲-: [$^3$H] labelled compound were used. Equal or higher sensitivity was found when using [$^{125}$I] labelling as compared with [$^3$H] labelling. Especially at low concentration (10 pg/Tube) higher sensitivity was observed.

(3) Comparison with mono-iodine [$^{125}$I] labelling and di-iodine [$^{125}$I] labelling :

Mono-iodine [$^{125}$I] labelling and di-iodine [$^{125}$I] labelling were compared according to the same method hereinabove. The result is shown in Fig. 3. In Fig. 3, -●- ●-: mono-iodine [$^{125}$I] labelling, and - ■- ■- : di-iodine [$^{125}$I] labelling.

The iodine radioisotope labelled 25-hydroxy vitamin $D_3$ derivative is of formula [V]:

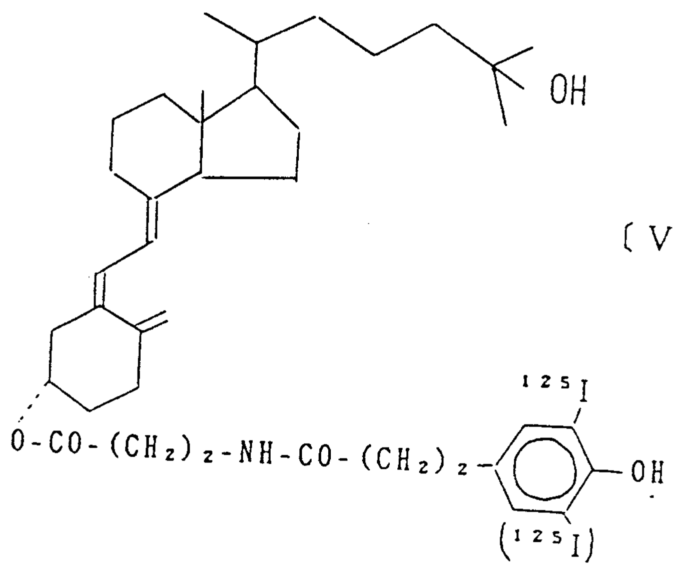

[V]

The assays using mono-iodine [$^{125}$I] labelled compound and di-iodine [$^{125}$I] labelled compound have almost the same sensitivity; mono-iodine [$^{125}$I] labelling is preferred.

(4) Comparison with methylene chain length in $R_1$ :

Assays using a compound of formula (IV) having $R_1$ = methylene [$-(CH_2)n-$ ] wherein n = 1, n = 2 or n = 3, were compared with each other.

The result is shown in Fig. 4. in which - ▲- ▲- : n = 1, - ●- ●- : n = 2 and - ■- ■- : n = 3. The n = 2 and n = 3, preferably n = 2, have good results as compared with n = 1.

Example 7 :

Evaluation of iodine radioisotope labelled 25-hydroxy vitamin $D_3$ by CPBA method using DBP in place of anti 25-hydroxy vitamin $D_3$ antibody :

(1) Preparation of DBP :

3.3 m ℓ fetal calf serum (FCS) diluted with phosphate buffer (pH 7.4, 0.01M) was charged on a column of Blue-Spharose (Tradename Pharmacia Corp. $\phi$ 2.5 X 7.5 cm) and de-albumin treatment through 40 m ℓ gel-bed volume was performed.

Fractions, each 5m ℓ, No. 7 - 10, checking with absorption at 280 nm were collected (total 20 m ℓ). FCS can instead be replaced by the serum of homeothermal animals.

(2) Standard curve of iodine [$^{125}$I] radioisotope labelled 25-hydroxy vitamin $D_3$ by CPBA-method using DBP, replaced for antibody :

1,024 pg/20$\mu$ ℓ standard ethanol solution of 25-hydroxy vitamin $D_3$ was diluted in a series manner by double in double dilution to prepare 32 pg/20$\mu$ ℓ, 16 pg/20 $\mu$ ℓ, 8 pg/20 $\mu$ ℓ , 4 pg/20 $\mu$ ℓ , 2 pg/20 $\mu$ ℓ and 1pg/20 $\mu$ ℓ solutions.

Samples (20$\mu$ ℓ) of each concentration hereinabove were pipetted into 2 tubes. 20 $\mu$l ethanol solution of iodine [$^{125}$I] radioisotope labeled 25-hydroxy vitamin $D_3$ derivative which contains antioxidant tocopherol was pipetted into each tube (20 $\mu$ ℓ ca. 30,000 cpm ). 500 $\mu$l DBP diluted with Tris buffer, pH 8.6 (approx. 500 ~ 1,000 dilution of DBP fraction in (1) hereinabove ) was stirred in each solution and allowed to stand at 4 °C for 16 hours.

Glycine buffer, pH 8.6 suspended with a mixture of dextran T70 and active charcoal (1 : 10), (each 300 $\mu$ ℓ) was added into the reaction tubes, stirred and incubated at 4 °C for 30 minutes. B-F separation was made by centrifugation at 3,000 r.p.m. for 10 minutes at 4°C, and each 500 $\mu$l supernatant solution was measured by an autowell $\tau$ -counter for 1 min. A binding ratio is caluculated by the following equation.

$$\text{Binding ratio} = \frac{(B) - (NSB)}{(Bo) - (NSB)} \times 100$$

wherein (B) is the count No. in each tube, (NSB) is the count No. measured by using 25-hydroxy vitamin $D_3$ (1024 pg/20 $\mu$ ℓ) instead of $^{125}$I labelled 25-hydroxy vitamin $D_3$ derivative and (Bo) is the count No. measured by using 25-hydroxy vitamin $D_3$ at zero concentration. Fig. 5 shows the standard curve of [$^{125}$I] labelled 25-hydroxy vitamin $D_3$ derivative.

(3) Stability of iodine [$^{125}$I] radioisotope labelled 25-hydroxy vitamin $D_3$ derivative :

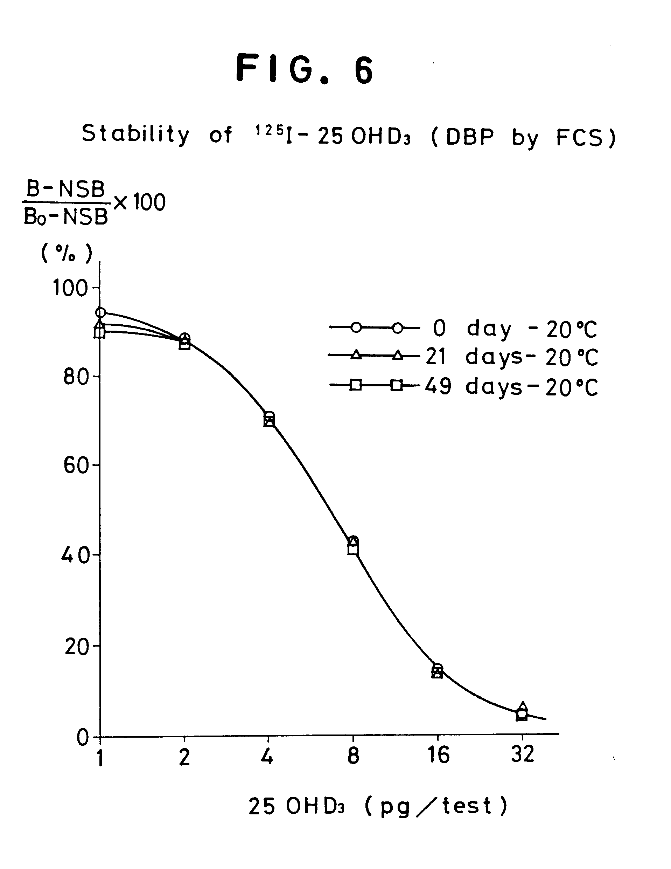

50 % $H_2O$-ethanol solution of [$^{125}$I] 25-hydroxy vitamin $D_3$ derivative obtained in Example 4 or Example 5 was stored at -20°C in an argon atomosphere in a vial. A standard curve using the above sample was prepared according to method (2) hereinabove. In Fig. 6 - ○- ○ - : day 0, -△- △- : day 21 and -□- □- : day 49 of standard curve.

As shown in Fig. 6, when it was stored at -20 °C, no changes of the standard curve within a range of 2 ~ 32 pg/Test were observed. The result shows that the [$^{125}$I] 25-hydroxy vitamin $D_3$ derivative can be used in a radio immunoassay with good stabililty.

Effect of invention :

As illustarated hereinabove, the present invention provides an iodine radioisotope labelled 25-hydroxy vitamin $D_3$ derivative with high radiation energy and widely applicability. Heretobefore, low radiation energy, $\beta$-ray emission tritium [$^3$H] labelled vitamin $D_3$ has been known. Until now, high radiation energy iodine [$^{125}$I] radioisotope vitamin $D_3$ was thought to degrade automatically due to a radical reaction on the conjugated double bond in the vitamin D structure. The [$^{125}$I] labelled 25-hydroxy vitamin $D_3$ derivative of the present invention does not undergo autodegradation by $\tau$-ray and is sufficiently stable for use in a radio immunoassay.

In general, vitamin $D_3$ is metabolized in vivo in the liver or kidney and converted to activated Vitamin $D_3$. The activated vitamin $D_3$, such as 25-hydroxy vitamin $D_3$, 1$\alpha$, 25 -dihydroxy vitamin $D_3$, 1$\alpha$-hydroxy vitamin $D_3$ and 1 $\alpha$,24 -dihydroxy vitamin $D_3$, is used clinically for the therapy of osteoporosis and osteomalacia. The clinical dose thereof is quite low due to its strong physicological action. Pharmacological activity is correlated to blood level and tissue level of the drug and hence measurement of blood level of the drug administered in humans is important from the clinical point of view. Considering the above the present invention is valuable in an assay of activated vitamin $D_3$ in clinical trials.

**Claims**

**1.** An iodine radioisotope labelled 25-hydroxy vitamin $D_3$ derivative of formula (III):

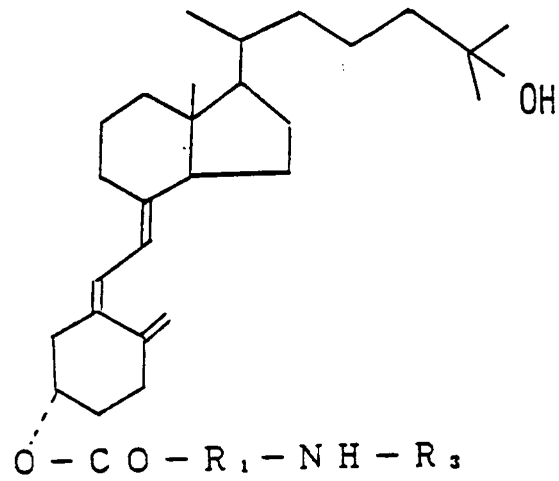

wherein $R_1$ is a $C_{1-10}$ alkylene group and $R_3$ is a group containing an iodine radioisotope.

**2.** An iodine radioisotope labelled 25-hydroxy vitamin $D_3$ derivative according to claim 1 wherein the iodine radioisotope is $^{125}I$.

**3.** An iodine radioisotope labelled 25-hydroxy vitamin $D_3$ derivative according to claim 2 wherein the group containing an iodine radioisotope is a 3-(4-hydroxy-3-iodo[$^{125}I$]-phenyl)-propionyl or 3-(3,5-diiodo-[$^{125}I$]-4-hydroxyphenyl)-propionyl group.

**4.** An iodine radioisotope labelled 25-hydroxy vitamin $D_3$ derivative according to any one of claims 1 to 3 wherein $R_1$ is a $C_{2-6}$ alkylene group.

**5.** An assay method of 25-hydroxy vitamin $D_3$ in a specimen which comprises:

adding, to a specimen containing 25-hydroxy vitamin $D_3$ to be assayed, an iodine radioisotope labelled 25-hydroxy vitamin $D_3$ derivative of formula (III) as defined in any one of claims 1 to 4;

adding anti 25-hydroxy vitamin $D_3$ antibodies to the specimen to bind competitively with the 25-hydroxy vitamin $D_3$ and the iodine radioisotope labelled 25-hydroxy vitamin $D_3$ derivative of formula (III);

separating the thus produced iodine radioisotope labelled 25-hydroxy vitamin $D_3$ derivative-anti 25-hydroxy vitamin $D_3$ antibody complex from the iodine radioisotope labelled 25-hydroxy vitamin $D_3$ derivative; and

measuring the amount of the iodine radioisotope label in bound or free form.

**6.** An assay method of 25-hydroxy vitamin $D_3$ in a specimen which comprises:

adding, to a specimen containing 25-hydroxy vitamin $D_3$ to be assayed, an iodine radioisotope labelled 25-hydroxy vitamin $D_3$ derivative of formula (III) as defined in any one of claims 1 to 4;

adding vitamin D binding protein to the specimen to bind competitively with the 25-hydroxy vitamin $D_3$ and the iodine radioisotope labelled 25-hydroxy vitamin $D_3$ derivative of formula (III);

separating the thus produced iodine radioisotope labelled 25-hydroxy vitamin $D_3$ derivative-vitamin D binding protein complex from the iodine radioisotope labelled 25-hydroxy vitamin $D_3$ derivative; and

measuring the amount of the iodine radioisotope in bound or free form.

**7.** A 25-hydroxy vitamin $D_3$ amino acid derivative of formula (I):

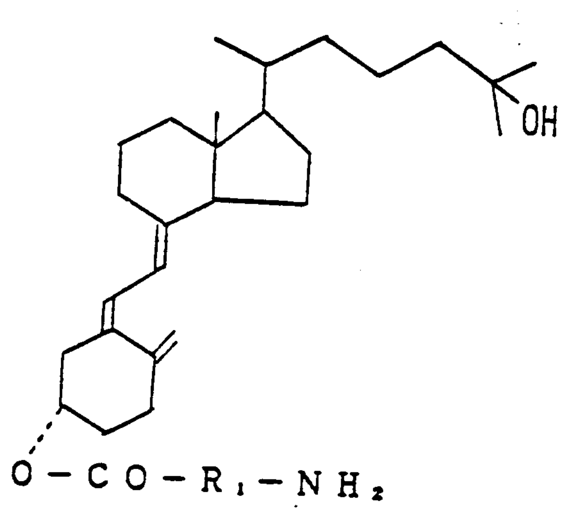

wherein $R_1$ is as defined in claim 1 or 4.

**8.** A process for preparing an iodine radioisotope labelled 25-hydroxy vitamin $D_3$ derivative of formula (III) as defined in any one of claims 1 to 4 wherein a 25-hydroxy vitamin $D_3$ derivative of formula (I) as defined in claim 7 is reacted with a compound of formula (IV):

$R_3$-X

wherein $R_3$ is as defined in any one of claims 1 to 3 and X is a succinimidyl-N-oxy, phthalimidyl-N-oxy, 5-norbornene-2,3-dicarboximidyl-N-oxy or maleimidyl-N-oxy group.

**9.** A process for preparing a 25 hydroxy vitamin $D_3$ amino acid derivative of formula (I) as defined in claim 7 wherein a compound of formula (II):

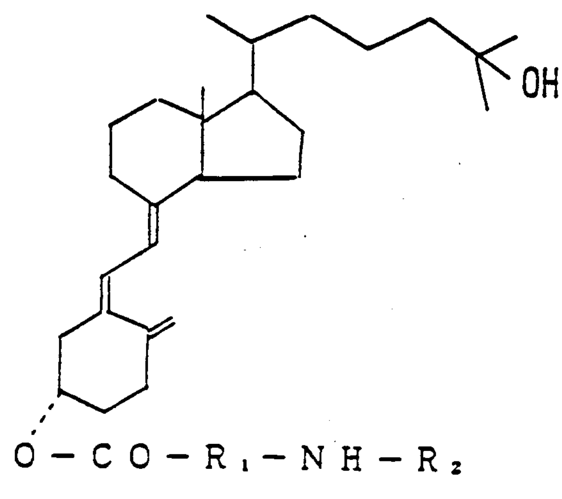

wherein $R_1$ is as defined in claim 1 or 4 and $R_2$ is an amino-protecting group, is deprotected in the presence of a base and in an inert solvent.

**10.** A process according to claim 9 wherein $R_2$ is a 9-fluorenyl methyloxycarbonyl group.

## Revendications

**1.** Dérivé de 25-hydroxy vitamine $D_3$ marqué par un radio-isotope d'iode de la formule (III) :

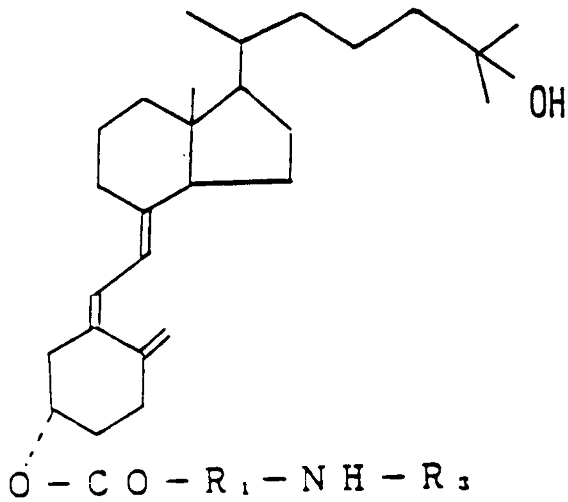

dans laquelle $R_1$ est un groupe alcoylène à 1 à 10 atomes de carbone et $R_3$ est un groupe contenant un radio-isotope d'iode.

**2.** Dérivé de 25-hydroxy vitamine $D_3$ marqué par un radio-isotope d'iode selon la revendication 1, dans lequel le radio-isotope d'iode est l'iode 125 ($^{125}$I).

**3.** Dérivé de 25-hydroxy vitamine $D_3$ marqué par un radio-isotope d'iode selon la revendication 2, dans lequel le groupe contenant un radio-isotope d'iode est un groupe 3-(4-hydroxy-3-iodo[$^{125}$I]-phényl)-propionyle ou un groupe 3-(3,5-diiodo[$^{125}$I]-4-hydroxyphényl)-propionyle.

**4.** Dérivé de 25-hydroxy vitamine $D_3$ marqué par un radio-isotope d'iode selon l'une des revendications 1 à 3, dans lequel $R_1$ est un groupe alcoylène à 2 à 6 atomes de carbone.

**5.** Méthode d'essai de la 25-hydroxy vitamine $D_3$ dans un échantillon, méthode qui consiste à :
ajouter, à un échantillon contenant la 25-hydroxy vitamine $D_3$ à essayer, un dérivé de 25-hydroxy vitamine $D_3$ marqué par un radio-isotope d'iode de la formule (III) comme défini dans l'une quelconque des revendications 1 à 4;
ajouter des anticorps anti 25-hydroxy vitamine 33 à l'échantillon pour se lier par compétition avec la 25-hydroxy vitamine $D_3$ et le dérivé de 25-hydroxy vitamine $D_3$ marqué par un radio-isotope d'iode de la formule (III);
séparer le complexe dérivé de 25-hydroxy vitamine $D_3$ marqué par un radio-isotope d'iode-anticorps anti 25-hydroxy vitamine $D_3$ ainsi produit du dérivé de 25-hydroxy vitamine $D_3$ marqué par un radio-isotope d'iode; et
mesurer la quantité du marqueur radio-isotope d'iode sous forme liée ou sous forme libre.

**6.** Méthode d'essai de la 25-hydroxy vitamine $D_3$ dans un échantillon, méthode qui consiste à :
ajouter, a un échantillon contenant la 25-hydroxy vitamine $D_3$ à essayer, un dérivé de 25-hydroxy vitamine $D_3$ marqué par un radio-isotope d'iode de la formule (III), comme défini dans l'une quelconque des revendications 1 à 4;
ajouter une protéine de liaison de la vitamine D à l'échantillon pour se lier par compétition avec la 25-hydroxy vitamine $D_3$ et le dérivé de 25-hydroxy vitamine $D_3$ marqué par un radio-isotope d'iode de la formule (III);
séparer le complexe dérivé de 25-hydroxy vitamine $D_3$ marqué par un radio-isotope d'iode-protéine de liaison de la vitamine D ainsi produit du dérivé de 25-hydroxy vitamine $D_3$ marqué par un radio-isotope d'iode; et
mesurer la quantité du radio-isotope d'iode sous forme liée ou sous forme libre.

**7.** Dérivé acide aminé de 25-hydroxy vitamine $D_3$ de la formule (I) :

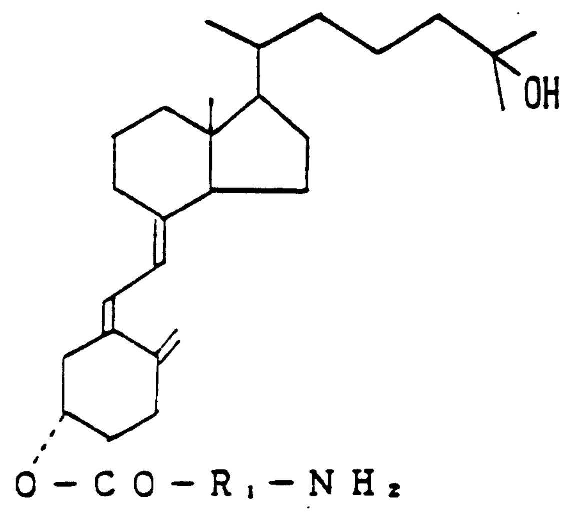

dans laquelle $R_1$ est comme défini dans la revendication 1 ou la revendication 4.

**8.** Procédé pour préparer un dérivé de 25-hydroxy vitamine $D_3$ marqué par un radio-isotope d'iode de la formule (III) comme défini dans l'une quelconque des revendications 1 à 4, dans lequel on fait réagir un dérivé de 25-hydroxy vitamine $D_3$ de la formule (I), comme défini dans la revendication 7, avec un composé de la formule (IV) :

$R_3$-X

dans laquelle $R_3$ est comme défini dans l'une quelconque des revendications 1 à 3 et X est un groupe succinimidyl-N-oxy, phtalimidyl-N-oxy, 5-norbornène-2,3-dicarboxymidyl-N-oxy ou maléimidyl-N-oxy.

**9.** Procédé pour préparer un dérivé d'acide aminé de 25-hydroxy vitamine $D_3$ de la formule (I) comme défini dans la revendication 7, dans lequel un composé de la formule (II) :

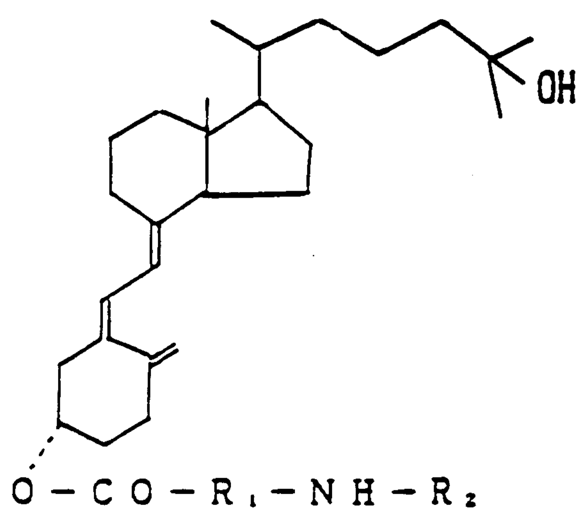

dans laquelle $R_1$ est comme défini dans la revendication 1 ou 4 et $R_2$ est un groupe de protection du groupe aminé, est déprotégé en la présence d'une base et dans un solvant inerte.

**10.** Procédé selon la revendication 9, dans lequel $R_2$ est un groupe 9-fluorényl méthyloxycarbonyle.

**Patentansprüche**

1. Iod-Radioisotop markiertes 25-Hydroxy-Vitamin-$D_3$-Derivat der Formel (III):

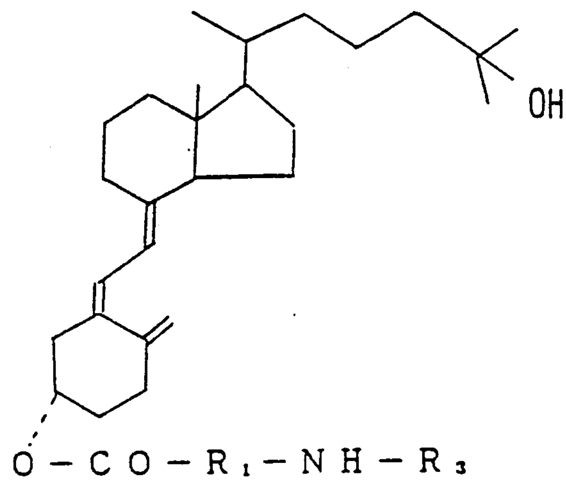

wobei $R_1$ eine $C_{1-10}$ Alkylengruppe und $R_3$ eine ein Iod-Radioisotop enthaltende Gruppe bedeutet.

2. Iod-Radioisotop markiertes 25-Hydroxy-Vitamin-$D_3$-Derivat nach Anspruch 1, wobei das Iod-Radioisotop $^{125}I$ ist.

3. Iod-Radioisotop markiertes 25-Hydroxy-Vitamin-$D_3$-Derivat nach Anspruch 2, wobei die ein Iod-Radioisotop enthaltende Gruppe eine 3-(4-Hydroxy-3-iodo[$^{125}I$]-phenyl)-propionyl- oder 3-(3,5-Diiodo[$^{125}I$)-4-hydroxyphenyl)-propionylgruppe bedeutet.

4. Iod-Radioisotop markiertes 25-Hydroxy-Vitamin-$D_3$-Derivat nach einem der Ansprüche 1 bis 3, wobei $R_1$ eine $C_{2-6}$ Alkylengruppe bedeutet.

5. Bestimmungsverfahren für 25-Hydroxy-Vitamin-$D_3$ in einer Probe, bei der man:
einer Probe, die zu bestimmendes 25-Hydroxy-Vitamin-$D_3$ enthält, ein Iod-Radioisotop-markiertes 25-Hydroxy-Vitamin-$D_3$-Derivatder Formel (III) gemäß Definition nach einem der Ansprüche 1 bis 4 zugibt;
der Probe anti-25-Hydroxy-Vitamin-$D_3$-Antikörper zusetzt, um sie kompetitiv mit dem 25-Hydroxy-Vitamin-$D_3$ und dem Iod-Radoisotop-markierten 25-Hydroxy-Vitamin-$D_3$-Derivat der Formel (III) zu binden;
den so erzeugten Iod-Radioisotop-markiertes 25-Hydroxy-Vitamin-$D_3$-Derivat/anti-25-Hydroxy-Vitamin-$D_3$-Antikörper-Komplex vom Iod-Radioisotop-markierten 25-Hydroxy-Vitamin-$D_3$-Derivatabtrennt; und
die Menge der Iod-Radioisotop-Markierung in freier oder gebundender Form mißt.

6. Bestimmungsverfahren für 25-Hydroxy-Vitamin-$D_3$ in einer Probe, bei der man:
einer Probe, die zu bestimmendes 25-Hydroxy-Vitamin-$D_3$ enthält, ein Iod-Radioisotop-markiertes 25-Hydroxy-Vitamin-$D_3$-Derivatder Formel (III) gemäß Definition nach einem der Ansprüche 1 bis 4 zugibt;
der Probe Vitamin D bindendes Protein zusetzt, um es kompetitiv mit dem 25-Hydroxy-Vitamin-$D_3$ und dem Iod-Radoisotop-markierten 25-Hydroxy-Vitamin-$D_3$-Derivat der Formel (III) zu binden;
den so erzeugten Iod-Radioisotop markiertes 25-Hydroxy-Vitamin-$D_3$-Derivat/Vitamin D bindendes Protein-Komplex vom Iod-Radioisotop-markierten 25-Hydroxy-Vitamin-$D_3$-Derivatabtrennt; und
die Menge der Iod-Radioisotop-Markierung in freier oder gebundender Form mißt.

7. 25-Hydroxy-Vitamin-$D_3$-Aminosäurederivat der Formel (I):

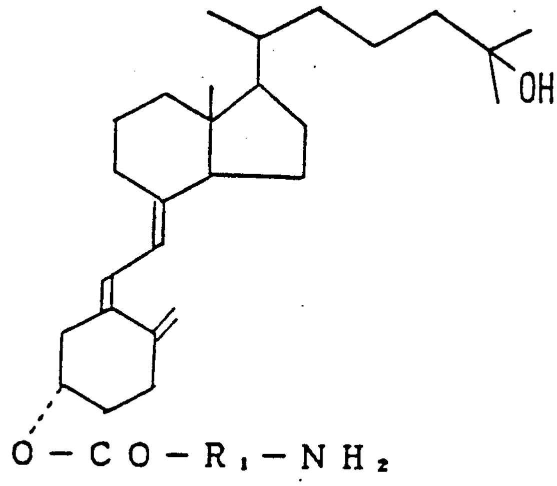

wobei $R_1$ wie in Anspruch 1 oder 4 definiert ist.

**8.** Verfahren zum Herstellen eines Iod-Radioisotop-markierten 25-Hydroxy-Vitamin-$D_3$-Derivats der Formel (III) gemäß Definition nach einem der Ansprüche 1 bis 4, wobei man ein 25-Hydroxy-Vitamin-$D_3$-Derivat der Formel (I) gemäß Definition in Anspruch 7 mit einer Verbindung der Formel (IV) umsetzt:

$R_3$-X,

wobei $R_3$ gemäß einem der Ansprüche 1 bis 3 definiert ist und X eine Succinimidyl-N-oxy-, Phthalimidyl-N-oxy-, 5-Norbornen-2,3-dicarboxyimidyl-N-oxy- oder Maleimidyl-N-oxy-Gruppe bedeutet.

**9.** Verfahren zum Herstellen eines 25-Hydroxy-Vitamin-$D_3$-Aminosäurederivats der Formel (I) gemäß Definition in Anspruch 7, wobei man von einer Verbindung der Formel (II):

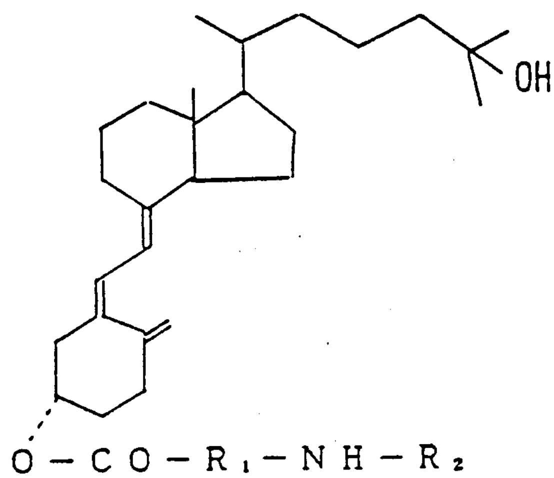

wobei $R_1$ gemäß Anspruch 1 oder 4 definiert ist und $R_2$ eine aminoschützende Gruppe bedeutet, in Gegenwart einer Base und in einem inerten Lösemittel die Schutzgruppe entfernt.

**10.** Verfahren nach Anspruch 9, wobei $R_2$ eine 9-Fluorenylmethyloxycarbonylgruppe bedeutet.

FIG. 1

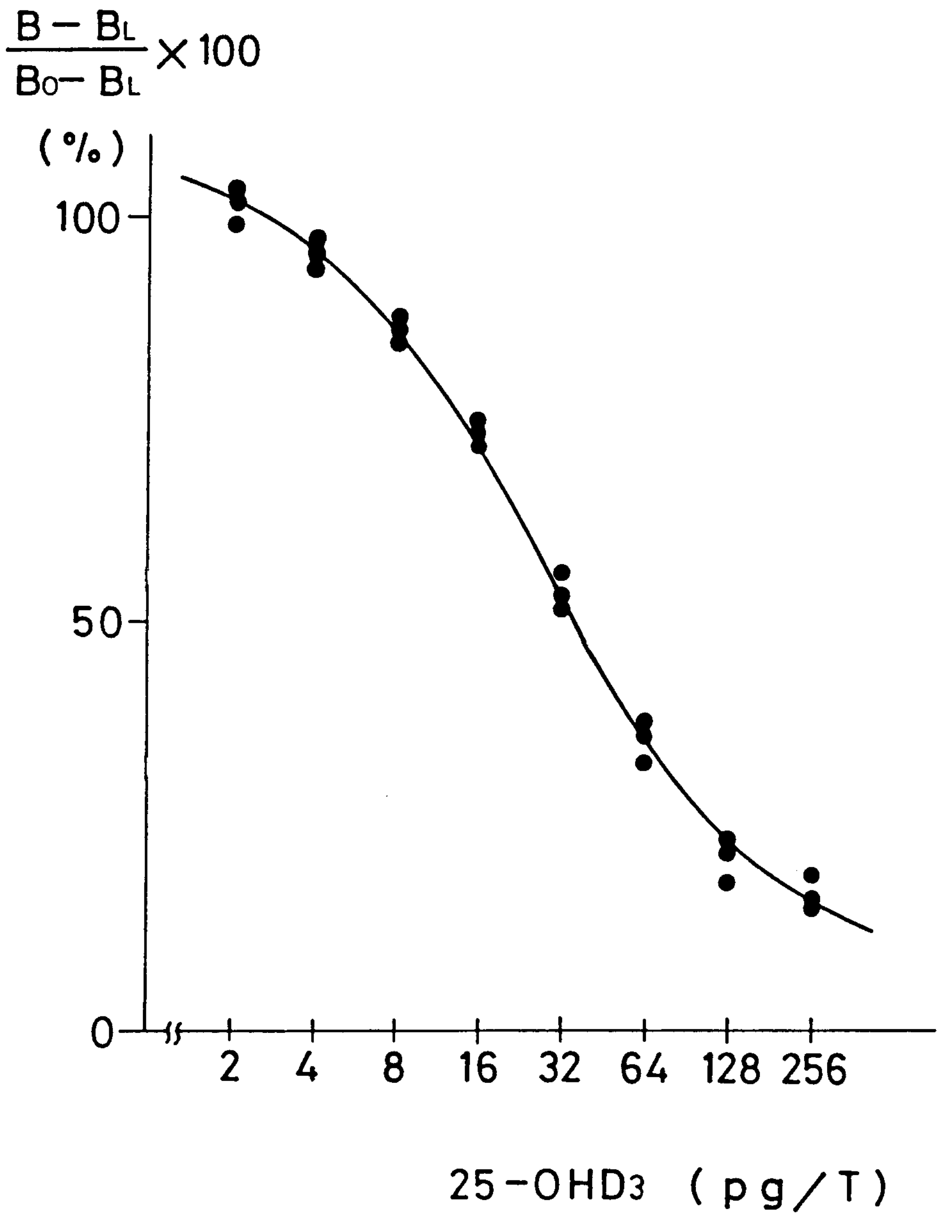

FIG. 2

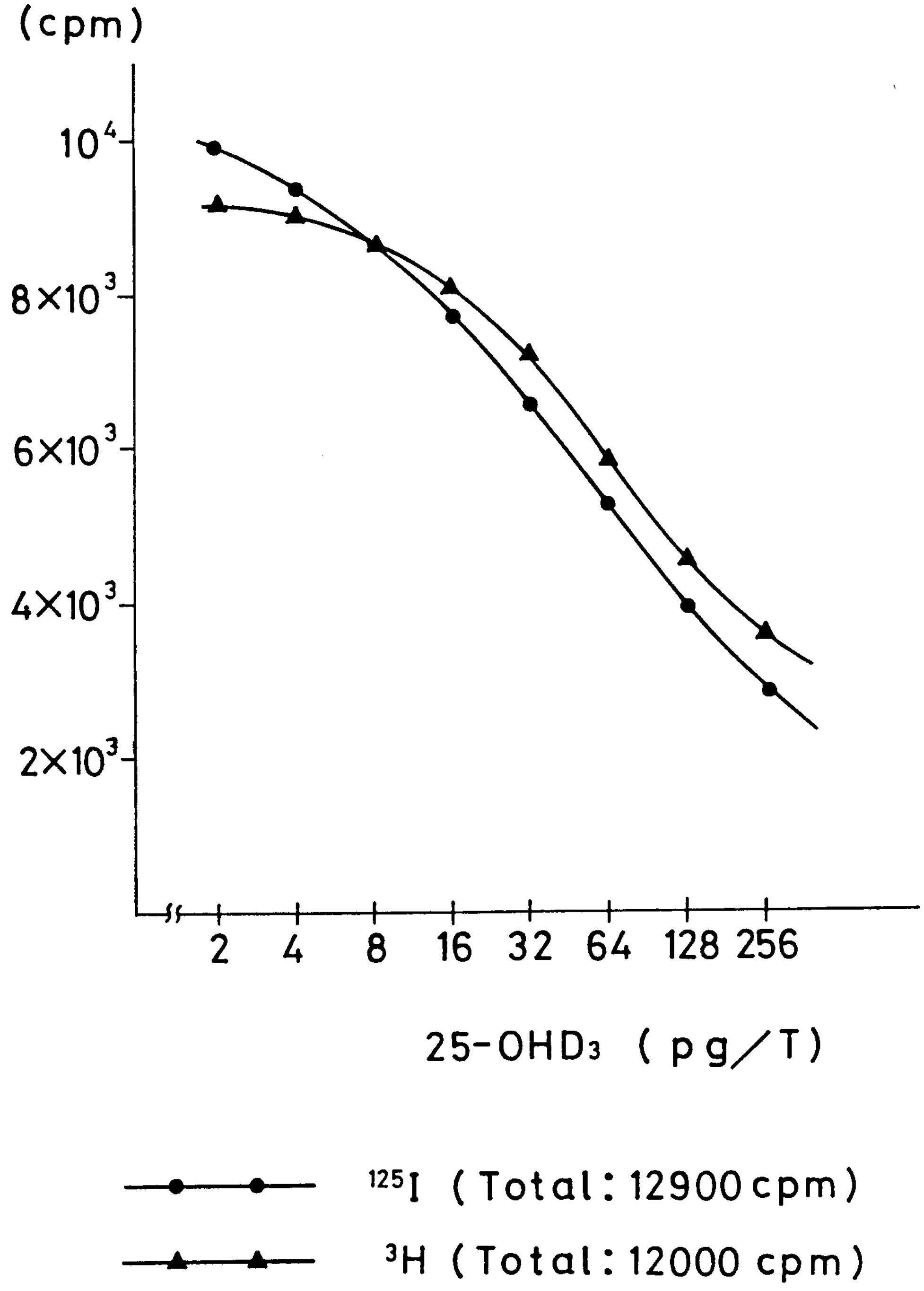
(cpm)
10⁴
8×10³
6×10³
4×10³
2×10³
2
4
8
16
32
64
128
256
25-OHD3 (pg/T)
125I (Total: 12900 cpm)
3H (Total: 12000 cpm)

# FIG. 3

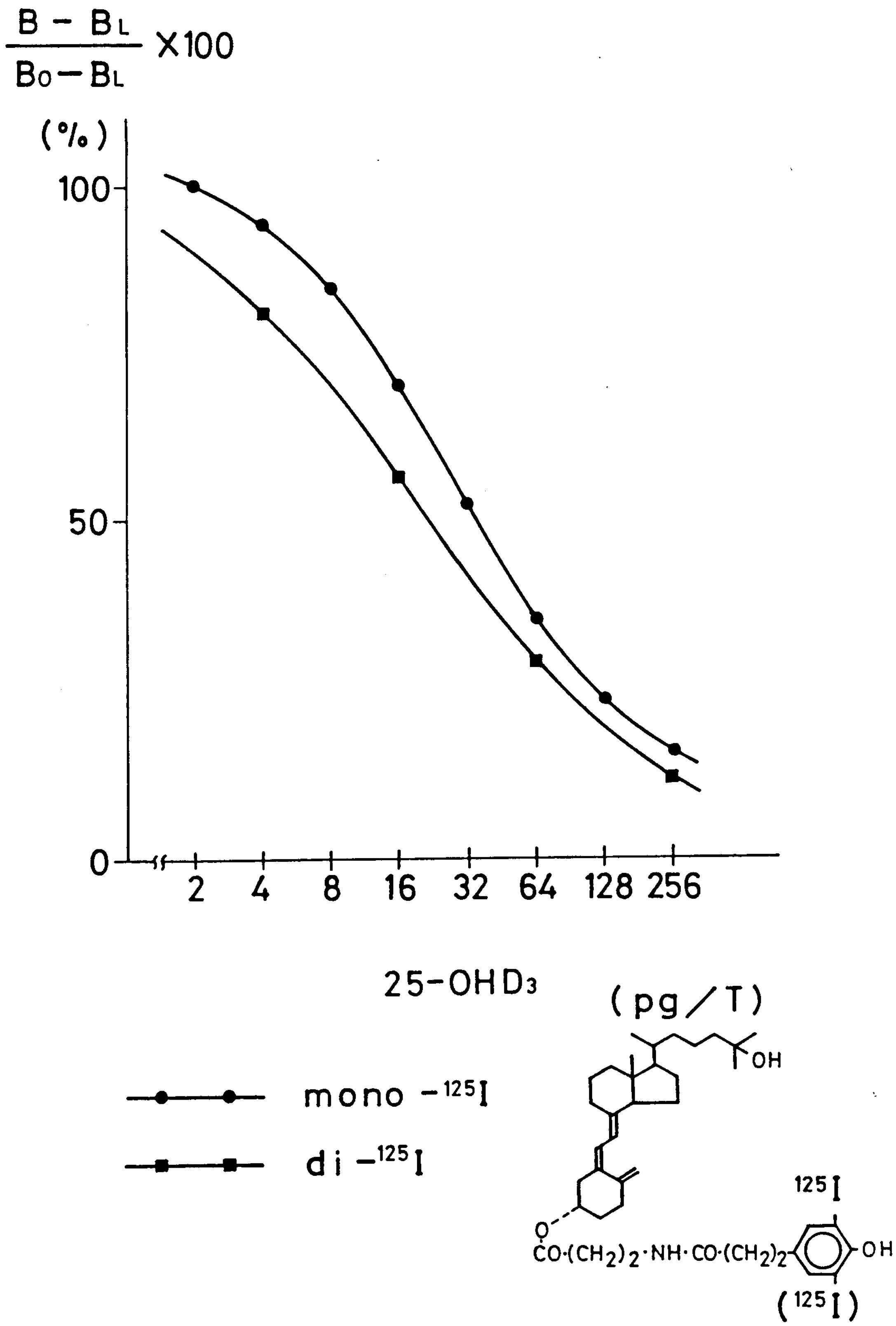

(B - BL)/(Bo - BL) X100
(%)
100
50
0
2
4
8
16
32
64
128
256
25-OHD3
(pg/T)
mono -125I
di -125I

FIG. 4

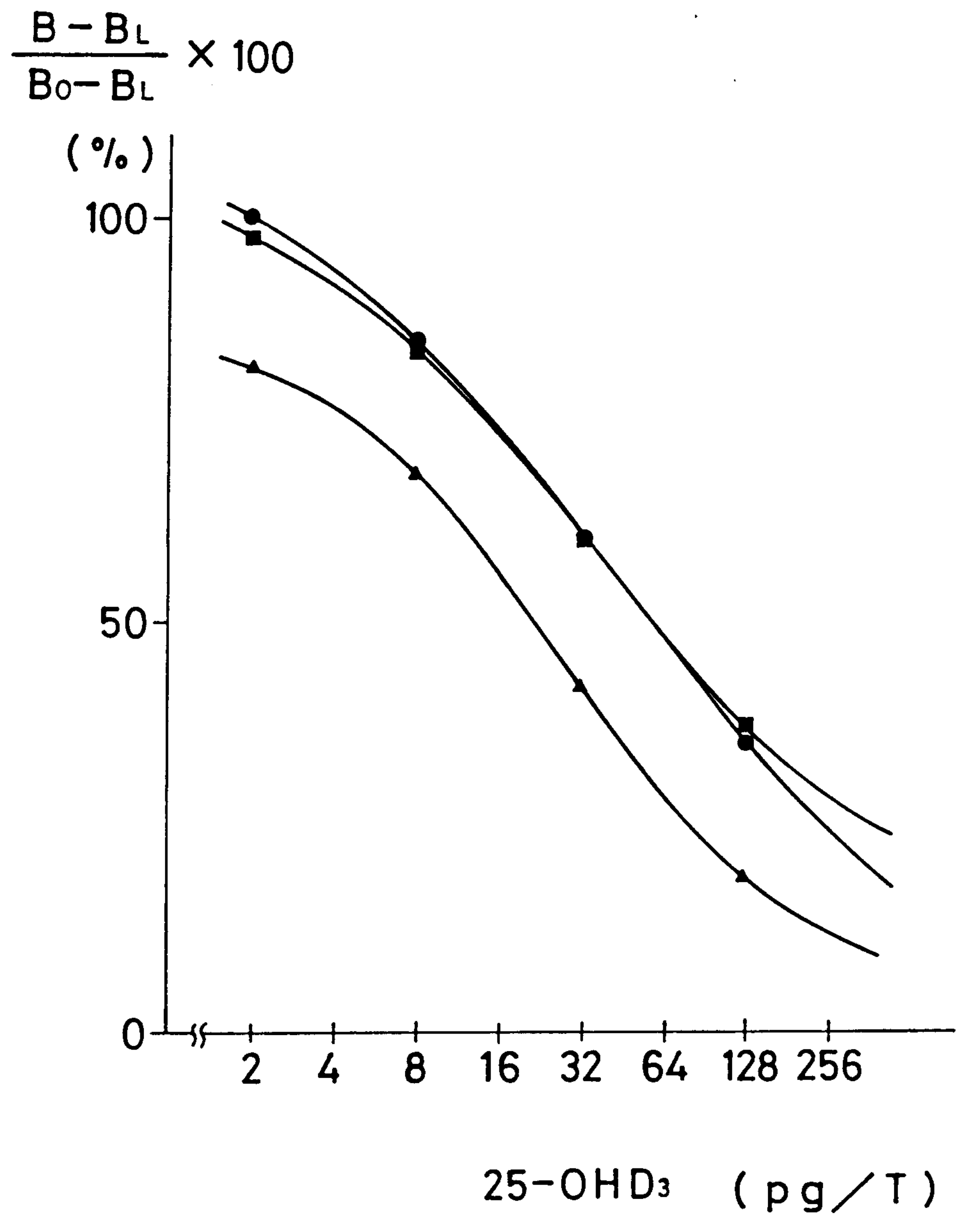


n = 1

n = 2

n = 3

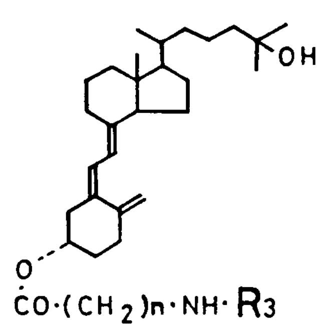

FIG. 5

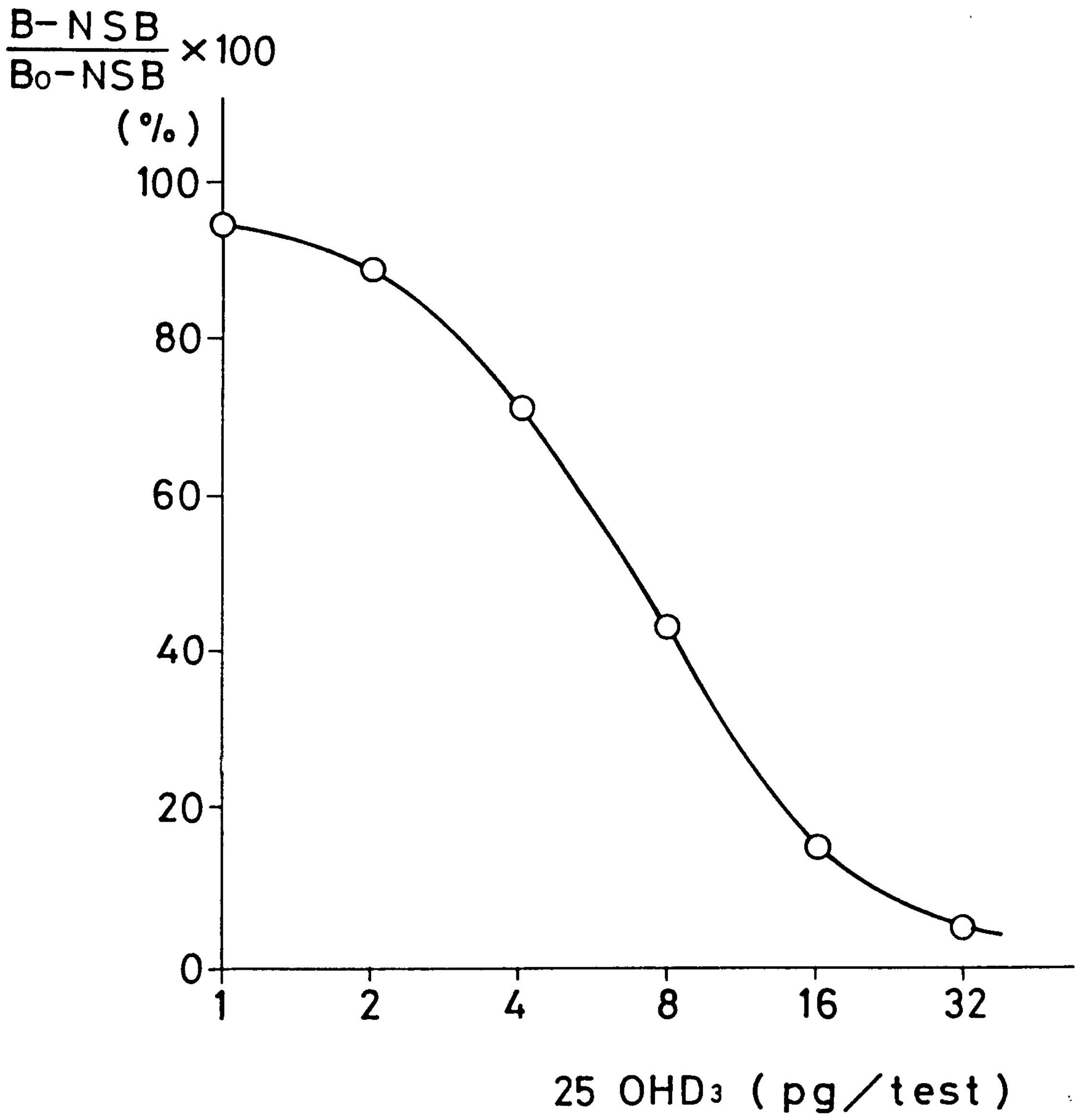

# FIG. 6

Stability of $^{125}I$-25 $OHD_3$ (DBP by FCS)